(19) Europäisches Patentamt
European Patent Office
Office européen des brevets

(11) Veröffentlichungsnummer: 0 367 256
A2

# (12) EUROPÄISCHE PATENTANMELDUNG

(21) Anmeldenummer: 89120268.1

(51) Int. Cl.5: C07C 59/135 , C07C 51/58

(22) Anmeldetag: 02.11.89

(30) Priorität: 04.11.88 DE 3837506

(43) Veröffentlichungstag der Anmeldung:
09.05.90 Patentblatt 90/19

(84) Benannte Vertragsstaaten:
DE FR GB IT NL

(71) Anmelder: HOECHST AKTIENGESELLSCHAFT
Postfach 80 03 20
D-6230 Frankfurt am Main 80(DE)

(72) Erfinder: von Werner, Konrad, Dr.
Wehrstrasse 6
D-8268 Garching(DE)

## (54) Verfahren zur Herstellung von Oligomeren des Hexafluorpropenoxids.

(57) Es wird ein Verfahren zur Herstellung von Oligomeren des Hexafluorpropenoxids durch Umsetzung von Hexafluorpropenoxid bei -60 bis +60 °C in Gegenwart einer Katalysatormischung beschrieben, die aus katalytischen Mengen von mindestens einem Fluorid eines ein- bis dreiwertigen Metalles und mindestens einem aprotischen tertiären Amin besteht. Die Katalysatormischung wird in polaren, aprotischen Lösungsmitteln, wie Nitrilen oder Ethern, angewendet. Das neue Verfahren ermöglicht die Herstellung von Oligomeren, die aus 3 bis 5 Einheiten des Hexafluorpropens aufgebaut sind, mit guten Ausbeuten, günstigen Reaktionszeiten und leicht einzuhaltenden Reaktionsbedingungen.

EP 0 367 256 A2

# Verfahren zur Herstellung von Oligomeren des Hexafluorpropenoxids

Die Erfindung betrifft ein Verfahren zur Herstellung von Oligomeren des Hexafluorpropenoxids nach Patentanspruch 1.

Für die Herstellung von Oligomeren des Hexafluorpropenoxids, nachfolgend HFPO genannt, sind eine Reihe von Verfahren bekannt. Aus einem zusammenfassenden Referat von Millauer, Schwertfeger und Siegemund, Angewandte Chemie, 97 (1985), Seiten 164 bis 182, insbesondere Seite 168, geht hervor, daß ein großer Teil dieser Verfahren die Herstellung des aus 2 HFPO-Einheiten entstandenen Säurefluorids zum Gegenstand hat. Dieses Produkt hat als Vorstufe zur Herstellung von Perfluorpropyl-perfluorvinylether, einem wichtigen Comonomeren in der Chemie der fluorierten Polymeren, erhebliche Bedeutung erlangt.

Es ist jedoch auch die Herstellung solcher Säurefluoride interessant, die aus 3 bis 5 HFPO-Einheiten gebildet werden, da sie beispielsweise als Vorprodukte für wirksame Fluortenside dienen. Zur Herstellung der tri- bis pentameren Derivaten des HFPO sind bisher nur Verfahren bekannt, die mindestens einen, bisweilen auch mehrere der folgenden Mängel aufweisen:

a) Schwerpunkt bei dimeren beziehungsweise trimeren Verbindungen, schlechte Ausbeute bei höheren Oligomeren (Tetramere beziehungsweise Pentamere);

b) breite Molmassen-Verteilung der erzeugten Oligomere, dadurch ebenfalls mäßige Ausbeuten im Bereich Trimeres bis Pentameres;

c) träge oder unvollständige Reaktion und damit schlechte Raum-Zeit-Ausbeuten;

d) vergleichsweise schnelle Inaktivierung des verwendeten Katalysatorsystems;

e) vermehrte Bildung unerwünschter, teilweise stark giftiger Nebenprodukte.

Aus der obengenannten Literaturstelle (Seiten 168 bis 169) ist bekannt, HFPO unter anderem mit Kaliumfluorid in Acetonitril als Lösungsmittel im Autoklaven während 6 Stunden bei +22 °C umzusetzen. Hierbei läßt die Ausbeute an Tetrameren und Pentameren des HFPO zu wünschen übrig. Die Verwendung eines Autoklaven läßt darauf schließen, daß trotz der vergleichsweise hohen Reaktionstemperatur die Oligomerisierung träge verläuft, da offenbar ständig ein Überdruck von HFPO, das nicht reagiert hat, verbleibt. Siehe hierzu auch den weiter unten beschriebenen Vergleichsversuch A.

Aus DE-OS 15 20 527 ist es bekannt, HFPO bei Temperaturen von -80 bis +50 °C mit einem System aus Katalysator plus Verdünnungsmittel umzusetzen. Als Katalysator sind unter anderem entweder Fluoride einwertiger Metalle, wie KF, CsF, AgF oder TlF, dispergiert in organischem Lösungsmittel, oder tertiäre Amine beziehungsweise tertiäre Aminoxide in organischen Lösungsmitteln beschrieben. Mit dem Metallfluorid-Katalysatoren werden Gemische von Produkten erhalten, die einen Anteil des Dimeren neben einem Gemisch von Produkten, die aus 3 bis 8 HFPO-Einheiten aufgebaut sind, oder HFPO-Polymerisate höheren Polymerisationsgrades enthalten. Nähere Angaben über die Kettenlängen-Verteilung fehlen. Sofern tertiäre Amine als Katalysator verwendet werden, entsteht, beispielsweise mit Dimethylanilin, das HFPO-Dimere als Hauptprodukt, während mit Pyridin ein Gemisch von Produkten, die aus 20 bis 25 HFPO-Einheiten aufgebaut sind, erhalten wird.

Nach dem in JP-OS 62-175 437 beschriebenen Verfahren wird zur Oligomerisierung von HFPO ein Katalysator-Gemisch, bestehend aus tertiärem Amin und einem Harnstoff- beziehungsweise Thioharnstoff-Derivat in aprotischen Lösungsmitteln eingesetzt. Hierbei wird mit hoher Selektivität das Dimere des HFPO gebildet, während das Trimere und Tetramere sowie höhere Oligomere nur in untergeordnetem Maße entstehen.

Ferner ist es aus JP-OS 62-195 345 bekannt, HFPO mit einem Katalysator-Gemisch zu oligomerisieren, das neben einem bekannten Katalysator eine Monohydroxy-Verbindung, wie Wasser, aliphatische Alkohole oder Phenole, oder ein sekundäres beziehungsweise protisches tertiäres Amin enthält, das mindestens einen Substituenten trägt, der wenigstens ein polar gebundenes Wasserstoffatom enthält. Durch Wahl eines geeigneten Verhältnisses von Katalysator und einem der obengenannten protischen Zusatzstoffe können die Mengenverhältnisse der verschiedenen HFPO-Oligomeren verändert werden. Es wird beispielsweise eine recht gute Ausbeute an Oligomeren, die aus 3, 4 und 5 HFPO-Einheiten aufgebaut sind, erhalten, wenn als Katalysator eine Mischung eingesetzt wird, die auf 1 mol Cäsiumfluorid 0,2 mol Wasser enthält. Der weiter unten beschriebene Vergleichsversuch E, bei dem anstelle von Wasser ein protisches tertiäres Amin verwendet wurde, zeigt jedoch, daß damit die genannten Oligomeren in wirtschaftlich vertretbaren Reaktionszeiten nicht erhalten werden können.

Die Aufgabe der vorliegenden Erfindung ist es, ein Verfahren bereitzustellen, mit dem bei vergleichsweise hohen Reaktionsgeschwindigkeiten Oligomere des HFPO hergestellt werden können, die bei enger Kettenlängenverteilung gute Ausbeuten an Produkten ergeben, die aus 3, 4 und 5 HFPO-Einheiten aufgebaut sind.

Diese Aufgabe wird gelöst durch ein Verfahren zur Herstellung von Oligomeren des Hexafluorpropenoxids durch Umsetzung von Hexafluorpropenoxid in Gegenwart von katalytischen Mengen von Metallfluoriden in geeigneten polaren, aprotischen Lösungsmitteln bei Temperaturen von -60 bis +60 °C, welches dadurch gekennzeichnet ist, daß als Katalysator eine Mischung verwendet wird, die aus mindestens einem Fluorid eines ein- bis dreiwertigen Metalles und mindestens einem aprotischen tertiären Amin besteht, das 6 bis 25 C-Atome und ein oder mehrere tertiäre Stickstoffatome enthält, von denen jedes tertiäre Stickstoffatom mit mindestens zwei gesättigten, aliphatischen Kohlenwasserstoffresten verbunden ist, die ihrerseits mit Fluoratomen substituiert sein können und/oder Ethersauerstoffatome enthalten können, wobei jedoch tertiäre Amine, die nur ein N-Atom neben ausschließlich C- und H-Atomen enthalten, ausgeschlossen sind.

Geeignete Fluoride sind beispielsweise Lithiumfluorid, Rubidiumfluorid, Silberfluorid, Kupfer(II)fluorid, Magnesiumfluorid, Zinkfluorid, Nickelfluorid, Kobalt(III)fluorid, Eisen(II)fluorid, Eisen(III)fluorid und Chrom(III)-fluorid. Bevorzugt werden Cäsiumfluorid, Calciumfluorid oder Mangan(II)fluorid verwendet. Besonders gute Ergebnisse werden mit Kaliumfluorid erhalten. Es können Mischungen von Fluoriden ein-, zwei- oder dreiwertiger Metalle untereinander sowie auch Mischungen solcher Fluoride mit anderen Haliden, insbesondere den Chloriden der genannten Metalle, verwendet werden.

Die erfindungsgemäß als Katalysator einzusetzende Mischung enthält neben mindestens einem Fluorid eines ein- bis dreiwertigen Metalles mindestens ein aprotischen tertiäres Amin, das 6 bis etwa 25 C-Atome und ein oder mehrere tertiäre Stickstoffatome enthält, von denen jedes tertiäre Stickstoffatom mit mindestens zwei gesättigten aliphatischen Kohlenwasserstoffresten verbunden ist. Diese aliphatischen Kohlenwasserstoffreste können ihrerseits mit Fluoratomen substituiert sein und/oder Ethersauerstoffatome enthalten. Ein tertiäres Stickstoffatom ist ein solches, bei dem alle drei Valenzen mit Kohlenstoffatomen verbunden sind. Tertiäre Amine, die nur ein N-Atom neben ausschließlich C- und H-Atomen enthalten, führen im allgemeinen nicht zu erwünschten höheren Anteilen an Oligomeren, die aus 3, 4 und 5 HFPO-Einheiten bestehen; sie sind deswegen für die Zwecke der vorliegenden Erfindung wenig geeignet. Ein tertiäres aprotisches Amin mit weniger als 6 C-Atomen zeigt nicht die erwünschte erfindungsgemäße Wirkung, nach oben ist die Zahl der C-Atome im tertiären Amin dadurch begrenzt, daß keine zusätzlich günstigen Wirkungen beobachtet werden, die die höheren Kosten für solche Verbindungen rechtfertigen würden, verschiedentlich wird auch eine Abnahme der Aktivität bezüglich der Bildung von Oligomeren aus 3 bis 5 HFPO-Einheiten festgestellt, ebenso kann die zunehmende Schwerlöslichkeit solcher höhermolekularer Verbindungen stören, weshalb man aprotische tertiäre Amine mit mehr als 25 C-Atomen im allgemeinen nicht verwenden wird. Vorzugsweise werden aprotische tertiäre Amine mit 6 bis 15 C-Atomen eingesetzt. Gute Ergebnisse werden erhalten, wenn als aprotisches tertiäres Amin mindestens eine Verbindung verwendet wird, die zwei bis vier Dialkylaminogruppen aufweist. Ebenfalls vorteilhaft wird als tertiäres Amin mindestens eine Verbindung eingesetzt, die mindestens eine $-CH_2-CH_2-O-CH_2-CH_2-$ oder mindestens eine $-CH_2-CH_2-O-CF_2-CF_2$-Gruppe enthält.

Geeignete Verbindungen sind beispielsweise:

$N(CH_2CH_2-O-CH_2CH_2-OCH_3)_3$

$N(CH_2CH_2-O-CF_2CF_2H)_3$

$CH_3-N(CH_2CH_2-O-CF_2CF_2H)_2$

$(CH_3)_2N-CH_2CH_2-O-CF_2CF_2H$

$(CH_3)_2N-CH_2CH_2CH_2-O-CF_2CF_2H$

$(CH_3)_2N-CH_2-CH(OCH_3)-CH(OCH_3)-CH_2-N(CH_3)_2$

Wegen ihrer guten Wirkung und leichten Beschaffbarkeit sind besonders bevorzugt die Verbindungen

$(CH_3)_2N-CH_2CH_2-N(CH_3)_2$

$(CH_3)_2N-CH_2CH_2-N(CH_3)-CH_2CH_2-N(CH_3)_2$

$(CH_3)_2N-CH_2CH_2-O-CH_2CH_2-N(CH_3)_2$.

Auf 1 mol Metallfluorid werden zweckmäßig 0,1 bis 10 mol aprotisches tertiäres Amin verwendet. Unter 0,1 mol tertiäres Amin je mol Metallfluorid verläuft im allgemeinen die Reaktion sehr langsam mit ungünstiger Raum-Zeit-Ausbeute, werden über 10 mol tertiäres Amin je mol Metallfluorid verwendet, so müssen entweder unnötig hohe Katalysatormengen eingesetzt werden oder es bilden sich zu wenig Oligomere, die aus 3 bis 5 HFPO-Einheiten bestehen, vorzugsweise werden je mol Metallfluorid 0,2 bis 4 und insbesondere 0,5 bis 2 mol aprotisches tertiäres Amin eingesetzt.

Die zur Umsetzung von 100 mol HFPO angewendete Menge Metallfluorid kann in weiten Grenzen schwanken, jedoch wird, wenn zu wenig Metallfluorid verwendet wird, eine deutliche Abnahme der Bildung von Oligomeren, die aus 4 und 5 HFPO-Einheiten bestehen, beobachtet. Vorzugsweise werden je 100 mol HFPO 0,01 bis 5 mol Metallfluorid eingesetzt. Prinzipiell können auch mehr als 5 mol Metallfluorid verwendet werden, jedoch tritt dadurch keine zusätzliche Wirkung mehr ein, die den erhöhten Kostenaufwand rechtfertigen würde. Gute Ergebnisse werden erhalten, wenn je 100 mol HFPO 0,1 bis 2 mol Metallfluorid angewendet werden.

Die Umsetzung des HFPO mit der erfindungsgemäßen Katalysatormischung wird in geeigeneten polaren, aprotischen Lösungsmitteln durchgeführt. Geeignet sind Ether, beispielsweise Dialkylether, Dioxan, Tetrahydrofuran, Dimethyl- oder Diethylether von Ethylenglykol und seinen Oligomeren, wie Diethylenglykol, Triethylenglykol oder Tetraethylenglykol. Besonders geeignet sind Nitrile von Carbonsäuren und Dinitrile von Dicarbonsäuren, wobei besonders gute Ergebnisse mit Acetonitril und Propionitril erzielt werden.

Die Menge Lösungsmittel, die je ein mol des in die Reaktion eingeführten HFPO angewendet wird, kann in weiten Grenzen schwanken. Im allgemeinen wird man 10 bis 1 000 $cm^3$, vorzugsweise 10 bis 300 $cm^3$ und insbesondere 20 bis 100 $cm^3$ Lösungsmittel je 1 mol HFPO anwenden.

Zweckmäßig wird vor Beginn der Reaktion eine Mischung aus Lösungsmittel, Metallfluorid und aprotischem, tertiärem Amin zübereitet, beispielsweise durch Rühren bei Zimmertemperatur während 10 bis 60 Minuten. Das Metallfluorid wird in feinteiliger Form eingesetzt.

Die Temperatur während der Umsetzung des HFPO beträgt -60 bis +60 °C. Unterhalb -60 °C wird die Reaktion zu langsam, auch stört im allgemeinen die zu hohe Viskosität des Reaktionsgemisches. Oberhalb +60 °C gerät die Reaktion leicht außer Kontrolle, es tritt eine vermehrte Bildung unerwünschter Nebenprodukte, beispielsweise von Perfluorpropionsäurefluorid ein. Vorzugsweise wird im Bereich von -20 bis +40 °C und insbesondere im Bereich von -5 bis +30 °C gearbeitet. Werden zwei- und/oder dreiwertige Metallfluoride eingesetzt, so empfiehlt es sich, im allgemeinen eine Temperatur von +10 °C nicht zu überschreiten, um eine gute Bildung von Oligomeren, die aus 4 beziehungsweise 5 HFPO-Einheiten aufgebaut sind, zu erreichen.

Das erfindungsgemäße Verfahren ist wenig druckabhängig, einer seiner Vorteile ist, daß bei Normaldruck gearbeitet werden kann. Es ist nicht erforderlich, den autogenen Druck des HFPO, beispielsweise bei Raumtemperatur (20 °C) einzuhalten, da durch die hohe Aktivität des erfindungsgemäßen Katalysatorgemisches beim Einleiten von HFPO mit hoher Reaktionsgeschwindigkeit dessen Oligomere gebildet werden, deren Dampfdruck bei Zimmertemperatur unterhalb des normalen Atmosphärendrucks (98,1 kPa) liegt.

Das erfindungsgemäße Verfahren kann kontinuierlich sowie auch absatzweise (chargenweise) durchgeführt werden. Die Reaktionszeit ist unterschiedlich; sie hängt von der Temperatur und der gewählten Katalysatormischung ab, übliche Reaktionszeiten sind 1 bis 10 Stunden.

Das Nachlassen der Wirkung des Katalysators kann leicht am Ansteigen der HFPO-Menge im Abgas erkannt werden. Durch Zugabe weiterer Mengen der erfindungsgemäßen Katalysatormischung kann die katalytische Aktivität wieder gesteigert und können weitere HFPO-Mengen umgesetzt werden.

Während der Reaktion des HFPO wird die Reaktionsmischung beispielsweise durch Rühren in Bewegung gehalten. Nach Beendigung der Zugabe von HFPO wird das Reaktionsgemisch vorteilhaft noch eine Zeit, beispielsweise 10 Minuten bis 2 Stunden, bei der gewählten Reaktionstemperatur weiter gerührt. Anschließend wird das Reaktionsgemisch, das zwei Phasen gebildet hat, beispielsweise in einem Scheidegefäß, getrennt. Die untere, schwerere Phase enthält die HFPO-Oligomeren. Sie kann beispielsweise durch fraktionierte Destillation weiter gereinigt werden. Oft kann das Rohprodukt ohne zusätzliche Reinigung zu weiteren Umsetzungen dienen, beispielsweise können durch Hydrolyse mit Eiswasser aus den Säurefluoriden die entsprechenden Carbonsäuren gewonnen werden. Die Carbonsäuren können dann ihrerseits in Salze, beispielsweise Ammoniumsalze, übergeführt werden.

Die erfindungsgemäße Reaktion wird unter Ausschluß von Wasser - zweckmäßig unter Verwendung eines trockenen Schutzgases, wie Stickstoff oder Argon - durchgeführt.

Achtung! Die aus dem HFPO entstehenden oligomeren Säurefluoride sind giftig und müssen unter entsprechenden Schutzmaßnahmen hergestellt und gehandhabt werden (Abzug, Schutzbrille, Handschuhe beziehungsweise Schutzkleidung).

Das erfindungsgemäße Verfahren eignet sich auch für die Oligomerisierung anderer aliphatischer, weitgehend fluorierter Epoxide der Formel

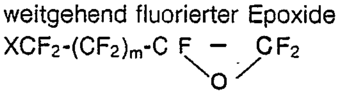

in der bedeuten X = F, H, Cl, Br oder

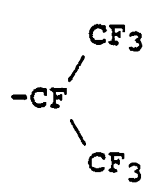

und m eine Zahl von 0 bis 12.

Durch das erfindungsgemäße Verfahren können oligomere Säurefluoride, die aus 3 bis 5 HFPO-Einheiten aufgebaut sind, unter günstig einzuhaltenden Reaktionsbedingungen mit guter Reaktionsgeschwindigkeit und Ausbeute erzeugt werden. Diese oligomeren Säurefluoride eignen sich als Vorprodukte beispielsweise für Anionentenside, die auch in hoher Verdünnung sehr niedrige Oberflächen- und Grenzflächenspannungen ergeben und interessante Emulgatoreigenschaften aufweisen, ferner als Vorprodukte für Sauerstoffträgerflüssigkeiten oder für hochtemperaturbeständige, chemisch inerte Flüssigkeiten.

Nachfolgende Beispiele und Vergleichsversuche sollen die Erfindung näher erläutern.

Es wird folgende Apparatur verwendet: Ein Edelstahlzylinder von 3 $dm^3$ Inhalt, in dem sich verflüssigtes HFPO befindet und der mit einem Druckreduzierventil ausgerüstet ist, steht auf einer Waage. Das Ventil ist vermittels einer Schlauchverbindung über einen Dreiwegehahn (zum Spülen mit trockenem Argon) mit einem Rotameter verbunden, das wiederum über einen weiteren Schlauch an einen Reaktor angeschlossen ist. Dieser Reaktor besteht aus einem zylindrischen Glasgefäß von 7,5 cm Durchmesser, 20 cm Höhe und circa 1 $dm^3$ Inhalt, hat einen ebenen Boden, eine außen angebrachte, vertikale Gaszuleitung mit Absperrhahn, die direkt über dem Boden in den Reaktor mündet, und ist am oberen Ende über mit Federzügen gesicherte Schliffverbindungen mit einem Thermometer und einem Trockeneiskühler ausgestattet. Der obere Ausgang des Kühlers ist mit einem federbelasteten Ventil verbunden, welches auf 200 kPa Überdruck eingestellt ist. Der Ausgang des Ventils ist nacheinander mit einem mit KOH-Pillen gefüllten Trockenrohr und einem mit Silikonöl gefüllten Blasenzähler verbunden. Der Reaktor steht in einer Glasschale mit ebenem Boden, die eine äußere Kühlung gestattet und die ihrerseits auf einer Magnetrühreinrichtung angeordnet ist.

Folgende Reagenzien werden verwendet: HFPO mit einem Gehalt von 99,5 Gew.-% HFPO, Rest Hexafluorpropen. Die Metallfluoride werden bei 200 °C unter einem Druck von 3 kPa während 2 bis 5 Stunden getrocknet und als feinteilige Pulver eingesetzt. Die polaren, aprotischen Lösungsmittel und die aprotischen tertiären Amine werden jeweils über einem Molekularsieb mit Porenweite 0,3 nm getrocknet.

## Beispiel 1

In den weiter oben beschriebenen, mit einem Polytetrafluorethylen beschichteten Magnetrührstab versehenen Reaktor werden unter trockenem Argon 1,16 g (0,02 mol) vorgetrocknetes Kaliumfluoridpulver gegeben und die Apparatur im Argonstrom mit einem Hochleistungsfön geheizt, um Spuren von Feuchtigkeit zu entfernen. Nun wird der Reaktor unter weiterer Einleitung von Argon abgekühlt und 100 $cm^3$ wasserfreies Acetonitril sowie 3,20 g (0,02 mol) 1,5-Bis-(dimethylamino)-3-oxapentan eingetragen. Die Katalysatormischung wird während 30 Minuten bei Raumtemperatur gerührt. Anschließend wird unter Fortsetzung des Rührens HFPO in die Katalysatormischung eingeleitet, während der ersten 15 Minuten in einer Menge von 10 $dm^3/h$, dann während weiterer 3 Stunden in einer Menge von 17,5 $dm^3/h$. Mittels einer Kältemischung im Kühlbad um den Reaktor und vermittels des Trockeneiskühlers wird die Temperatur des Reaktionsgemisches bei +20 ± 2 °C gehalten, insgesamt werden 405 g (2,4393 mol) HFPO eingetragen. Danach wird noch eine Stunde ohne Kühlung zur Nachreaktion weitergerührt. Das Reaktionsgemisch wird nun unter Argon in ein Scheidegefäß gegeben, in dem sich zwei übereinanderliegende flüssige Phasen abscheiden, die voneinander getrennt werden. Als untere, schwerere Phase werden 395 g eines flüssigen Rohproduktes (97,5 % Ausbeute, bezogen auf eingesetztes HFPO) erhalten, das im wesentlichen aus den HFPO-Oligomeren der Formel $CF_3CF_2\text{-}[CF_2\text{-}O\text{-}CF(CF_3)]_{n-1}\text{-}COF$, in der n die Zahl der HFPO-Einheiten, aus denen die Verbindung aufgebaut ist, angibt, besteht. Nach der weiter unten näher beschriebenen gaschromatischen Analyse besteht das Rohprodukt aus Verbindungen der genannten Formel mit folgender Oligomerenverteilung (Prozent = Flächenprozent): n = 2: 1,4 %; n = 3: 16,9 %; n = 4: 45,8 %; n = 5: 30,8 %; n = 6: 3,8 %; Rest (n unbekannt): 1,3 %. Aus dem $^{19}F$-NMR-Spektrum wird eine mittlere Molmasse $\overline{M}$ = 690 berechnet.

Die fraktionierte Destillation des Rohproduktes ergibt als farblose Flüssigkeiten folgende Hauptfraktionen, deren Reinheit nach gaschromatographischer Untersuchung 97 bis 99,5 % beträgt:

| HFPO-Einheiten n im Oligomeren | erhaltene Menge in g | Siedepunkt °C | Druck (kPa) |
|---|---|---|---|
| 2 | 5,2 | 56 | 1 010 |
| 3 | 62,3 | 116 | 1 010 |
| 4 | 174,5 | 98 - 99 | 100 |
| 5 | 118,3 | 130 | 100 |
| 6 | 11,4 | 114 | 15 |

Analog Beispiel 1 werden alle in nachfolgender Tabelle angegebenen Beispiele und Vergleichsversuche durchgeführt, wobei die Vergleichsversuche mit großen Buchstaben, die Beispiele mit arabischen Zahlen bezeichnet sind. Die Reaktionszeiten betrugen bei Beispiel 1: 4,25 Stunden, bei den Beispielen 2 bis 7 und 9 bis 15: 4 Stunden (3 Stunden Einleiten des HFPO und 1 Stunde Nachreaktion), bei Beispiel 8: 7 Stunden (6 Stunden Einleiten des HFPO und 1 Stunde Nachreaktion). Die Vergleichsversuche werden mit folgenden Reaktionszeiten durchgeführt: Versuch A: 10 Stunden, Versuch B: 4 Stunden, Versuch C: Es wird HFPO während 0,5 Stunden eingeleitet, dann hat sich bereits ein starker Rückfluß von nicht umgesetztem HFPO gebildet, weshalb die Einleitung beendet wird, während einer Nachreaktionszeit von 20 Stunden hat sich die Rückflußmenge wenig geändert, der Versuch wird abgebrochen; Versuch D: 4 Stunden, Versuch E: 23 Stunden.

In nachfolgender Tabelle I sind die Versuchsbedingungen und Ergebnisse für die Beispiele und Vergleichsversuche aufgeführt.

Fußnoten zu Tabelle I:

1) mol Amin:100 mol HFPO

2) Tetraglyme = Tetraethylenglykoldimethylether

3) $(CH_3)_2NCH_2CH(OCH_3)CH(OCH_3)CH_2N(CH_3)_2$

4) $(CH_3)_2NCH_2CH_2N(CH_3)CH_2CH_2N(CH_3)_2$

5) Analog JP-OS 62-195 345 mit einem protischen Amin anstelle von Wasser. In eine Mischung von 2,74 g Cäsiumfluorid (0,018 mol) plus 0,32 g $(CH_3)_2NCH_2CH_2OH$ (0,0036 mol) plus 8 g Tetraglyme werden während 8 Stunden 300 g HFPO (1,807 mol) eingegast, wobei sehr schnell Rückfluß durch nicht umgesetztes HFPO und monomeres Umlagerungsprodukt Perfluorpropionsäurefluorid eintritt. Nach Beendigung der Einleitung von HFPO wird das Reaktionsgemisch noch 15 Stunden weitergerührt. Auch nach dieser Zeit hat sich keine HFPO-Oligomeren enthaltende flüssige Phase abgeschieden. (Nach dem Beispiel der JP-OS 62-195 345 gab ein Zusatz von 0,0036 mol Wasser die beste Ausbeute an HFPO-Oligomeren.)

TABELLE I

| Beispiel | Metall-fluorid | mol Fluorid/ 100 mol HFPO | tertiäres Amin | mol Amin/ 1 mol Fluorid | Lösemittel | ccm/mol HFPO | Temperatur °C |
|---|---|---|---|---|---|---|---|
| A | KF | 5,6 | - | - | $CH_3CN$ | 278 | +20 |
| B | KF | 1,6 | $N(C_2H_5)_3$ | 1 | $CH_3CN$ | 82 | +20 |
| C | KF | 8,3 | $(CH_3)_2NCH_2CH_2OH$ | 1 | $CH_3CN$ | 417 | +20 |
| D | - | 1,6 [1] | $(CH_3)_2NCH_2CH_2OCF_2CF_2H$ | - | $CH_3CN$ | 82 | +20 |
| E [5] | CsF | 1,0 | $(CH_3)_2NCH_2CH_2OH$ | 0,2 | Tetraglyme [2] | 4,4 | -20 |
| 1 | KF | 0,82 | $(CH_3)_2NCH_2CH_2OCH_2CH_2N(CH_3)_2$ | 1 | $CH_3CN$ | 41 | +20 |
| 2 | KF | 1,2 | $(CH_3)_2NCH_2CH_2N(CH_3)_2$ | 0,9 | $CH_3CN$ | 22 | +20 |
| 3 | KF | 2,0 | $(CH_3)_2NCH_2CH_2N(CH_3)_2$ | 1 | $CH_3CN$ | 81 | -5 |
| 4 | KF | 1,6 | $(CH_3)_2NCH_2CH_2OCF_2CF_2H$ | 1 | $CH_3CN$ | 82 | +20 |
| 5 | KF | 1,6 | $N(CH_2CH_2OCH_2CH_2OCH_3)_3$ | 1 | $CH_3CN$ | 82 | +20 |
| 6 | KF | 2,0 | 3) | 1 | $CH_3CN$ | 45 | +10 |
| 7 | KF | 1,6 | $(CH_3)_2NCH_2CH_2OCH_2CH_2N(CH_3)_2$ | 2 | $CH_3CN$ | 82 | +20 |
| 8 | KF | 3,3 | $N(CH_2CH_2OCF_2CF_2H)_3$ | 1 | $CH_3CN$ | 166 | +20 |
| 9 | KF | 1,6 | $(CH_3)_2NCH_2CH_2CH_2OCF_2CF_2H$ | 1 | $CH_3CN$ | 82 | +20 |
| 10 | KF | 2,0 | $(CH_3)_2NCH_2CH_2N(CH_3)_2$ | 1 | $CH_3CH_2CN$ | 81 | -5 |
| 11 | $CaF_2$ | 2,0 | $(CH_3)_2NCH_2CH_2N(CH_3)_2$ | 2,2 | $CH_3CN$ | 82 | -5 |
| 12 | CsF | 2,0 | $(CH_3)_2NCH_2CH_2N(CH_3)_2$ | 1 | $CH_3CN$ | 82 | -5 |
| 13 | $MnF_2$ | 2,0 | $(CH_3)_2NCH_2CH_2N(CH_3)_2$ | 2 | $CH_3CN$ | 82 | -5 |
| 14 | $MnF_2$ | 2,0 | 4) | 1,5 | $CH_3CN$ | 81 | -5 |
| 15 | $MnF_2$ | 2,0 | $(CH_3)_2NCH_2CH_2N(CH_3)_2$ | 2 | $CH_3CH_2CN$ | 81 | -5 |

TABELLE I (Fortsetzung)

| Beispiel | Ausbeute Gew.-% RCOF bezogen auf eingesetztes HFPO | Zusammensetzung des RCOF: $CF_3CF_2-[CF_2-O-CF(CF_3)]_{n-1}-COF$ Flächen-% in GC // n (Zahl der HFPO-Moleküle) = 2 | 3 | 4 | 5 | 6 | Rest | Erzeugte Verbindungen g/h RCOF n = 2 bis 6 + Rest |
|---|---|---|---|---|---|---|---|---|
| A | 76,6 | 0,3 | 8,7 | 34,0 | 41,4 | 8,5 | 6,1 | 2,3 |
| B | 86,4 | 68,1 | 27,5 | 1,8 | - | - | 2,6 | 21,8 |
| C | 15,0 | 6,5 | 8,0 | - | - | - | 84,5 | 0,2 |
| D | 89,1 | 43,4 | 47,4 | 8,2 | 0,3 | - | 0,7 | 22,5 |
| E 5) | 0 | - | - | - | - | - | - | - |
| 1 | 97,5 | 1,4 | 16,9 | 45,8 | 30,8 | 3,8 | 1,3 | 92,9 |
| 2 | 96,2 | 4,1 | 19,8 | 45,0 | 27,2 | 3,5 | 0,4 | 97,4 |
| 3 | 94,4 | 2,1 | 18,1 | 50,1 | 27,4 | 1,9 | 0,4 | 48,3 |
| 4 | 94,1 | 1,5 | 10,6 | 35,7 | 40,8 | 10,7 | 0,7 | 23,7 |
| 5 | 88,1 | 0,6 | 7,7 | 31,2 | 45,2 | 14,4 | 0,9 | 22,4 |
| 6 | 94,9 | 3,6 | 17,5 | 38,4 | 32,5 | 6,8 | 1,2 | 47,6 |
| 7 | 96,8 | 2,4 | 14,1 | 43,7 | 33,8 | 4,7 | 1,3 | 24,4 |
| 8 | 87,2 | 3,1 | 11,4 | 34,8 | 40,1 | 10,1 | 0,5 | 6,3 |
| 9 | 84,1 | 0,9 | 8,8 | 37,7 | 42,3 | 8,6 | 1,7 | 21,2 |
| 10 | 96,6 | 1,6 | 11,3 | 40,0 | 39,5 | 7,0 | 0,6 | 49,7 |
| 11 | 97,1 | 7,7 | 20,5 | 53,0 | 17,6 | 0,4 | 0,8 | 48,7 |
| 12 | 91,1 | 7,4 | 42,1 | 42,8 | 7,3 | 0,2 | 0,2 | 46,2 |
| 13 | 91,1 | 6,4 | 33,5 | 45,3 | 14,0 | 0,7 | 0,1 | 46,2 |
| 14 | 83,3 | 3,0 | 15,8 | 36,5 | 34,2 | 9,8 | 0,7 | 42,7 |
| 15 | 77,1 | 4,0 | 23,2 | 42,8 | 25,7 | 4,1 | 0,2 | 39,7 |

Die Zusammensetzung der HFPO-Oligomerisationsprodukte der Formel $CF_3CF_2-[CF_2-O-CF(CF_3)]_{n-1}-COF$ wird wie folgt ermittelt:

Durch direkte Gaschromatographie der Säurefluoride in einem F22-Perkin-Elmer-Chromatograph mit Stahlsäule von 4 m Länge und 0,2 cm Durchmesser, Trennphase: 10 % UCCB auf Embacel, Injektortemperatur 250 °C, Temperaturprogramm: 60 bis 250 °C bei einer Aufheizrate von 4 °C/min, Heliumströmung 20 $cm^3$/min, WLD-Detektor.

Die so erhaltenen Flächenprozent-Werte entsprechen in guter Näherung der Gewichtsverteilung der Einzelkomponenten.

Aus dem $^{19}$F-NMR-Spektrum der Säurefluoride kann ferner die mittlere Molmasse $\overline{M}$ bestimmt werden, als Relation des COF-Integrals zur Summe aller anderen Integrale, wenn man die Messung unter Ausschluß von Feuchtigkeit durchführt.

## Ansprüche

1. Verfahren zur Herstellung von Oligomeren des Hexafluorpropenoxids durch Umsetzung von Hexafluorpropenoxid in Gegenwart von katalytischen Mengen von Metallfluoriden in geeigneten polaren, aprotischen Lösungsmitteln bei Temperaturen von -60 bis +60 °C, dadurch gekennzeichnet, daß als Katalysator eine Mischung verwendet wird, die aus mindestens einem Fluorid eines ein- bis dreiwertigen Metalles und mindestens einem aprotischen tertiären Amin besteht, das 6 bis 25 C-Atome und ein oder mehrere tertiäre Stickstoffatome enthält, von denen jedes tertiäre Stickstoffatom mit mindestens zwei gesättigten, aliphatischen Kohlenwasserstoffresten verbunden ist, die ihrerseits mit Fluoratomen substituiert sein können und/oder Ethersauerstoffatome enthalten können, wobei jedoch tertiäre Amine, die nur ein N-Atom neben ausschließlich C- und H-Atomen enthalten, ausgeschlossen sind.

2. Verfahren nach Anspruch 1, dadurch gekennzeichnet, daß ein Fluorid von mindestens einem der folgenden Metalle verwendet wird: Kalium, Cäsium, Mangan(II), Calcium.

3. Verfahren nach Anspruch 1 oder 2, dadurch gekennzeichnet, daß als tertiäres Amin mindestens eine Verbindung eingesetzt wird, die 2 bis 4 Dialkylaminogruppen aufweist.

4. Verfahren nach Anspruch 1 oder 2, dadurch gekennzeichnet, daß als tertiäres Amin mindestens eine Verbindung eingesetzt wird, die mindestens eine $-CH_2-CH_2-O-CH_2-CH_2-$ oder mindestens eine $-CH_2-CH_2-O-CF_2-CF_2$-Gruppe enthält.

5. Verfahren nach einem oder mehreren der Ansprüche 1 bis 4, dadurch gekennzeichnet, daß als Katalysator eine Mischung eingesetzt wird, die auf 1 mol Metallfluorid 0,2 bis 4 mol tertiäres Amin enthält.

6. Verfahren nach einem oder mehreren der Ansprüche 1 bis 5, dadurch gekennzeichnet, daß je 100 mol Hexafluorpropenoxid 0,01 bis 5 mol Metallfluorid eingesetzt werden.

7. Verfahren nach einem oder mehreren der Ansprüche 1 bis 6, dadurch gekennzeichnet, daß die Umsetzung von Hexafluorpropenoxid mit der Katalysatormischung bei -20 bis +40 °C erfolgt.

8. Verfahren nach einem oder mehreren der Ansprüche 1 bis 7, dadurch gekennzeichnet, daß als polares, aprotisches Lösungsmittel mindestens eines der folgenden eingesetzt wird: Acetonitril, Propionitril.

9. Verfahren nach einem oder mehreren der Ansprüche 1 bis 8, dadurch gekennzeichnet, daß je 1 mol Hexafluorpropenoxid 10 bis 300 $cm^3$ Lösungsmittel eingesetzt werden.